# EUROPEAN PATENT SPECIFICATION

(11) **EP 1 517 690 B1**
(45) Date of publication and mention of the grant of the patent: **12.03.2014**
(21) Application number: 03742408.2
(22) Date of filing: 02.07.2003
(51) Int. Cl.: A61K 31/4439, A61K 9/08, A61K 9/10

(54) **LIQUID DOSAGE FORMS OF NON-ENTERICALLY COATED ACID-LABILE DRUGS**
FLÜSSIGE DARREICHUNGSFORMEN VON NICHT-ENTERISCHUMMANTELTEN, SÄURELABILEN ARZNEISTOFFEN
FORMES GALENIQUES LIQUIDES DE MEDICAMENTS LABILES EN MILIEU ACIDE PELLICULES NON GASTRO-RESISTANTS

(30) Priority: 03.07.2002 US 190240
(43) Date of publication of application: 30.03.2005
(73) Proprietor: Takeda Pharmaceuticals U.S.A., Inc., Deerfield, IL 60015 (US)
(72) Inventor: TANEJA, Rajneesh, Libertyville, IL 60048 (US)
(74) Representative: Modiano, Micaela Nadia
(86) International application number: PCT/US2003/020876
(87) International publication number: WO 2004/004719

(56) References cited:
- EP-A- 0 547 000
- WO-A-01/13897
- WO-A-01/51050
- WO-A-99/36060
- US-A- 5 589 491
- US-A- 5 731 002
- US-A- 5 840 737
- US-A1- 2002 192 299

## Description

### Technical Field

The present invention relates to liquid dosage forms and in particular, relates to liquid formulations of acid labile drugs.

### Background of the Invention

Proton pump inhibitors (or "PPIs") are a class of pharmaceutical compounds that inhibit gastric acid secretions by inhibiting H+/K+ adenosine triphosphate, an enzyme present in parietal cells found in the gastric lining of the stomach. H+/K+ adenosine triphosphate is variously referred to as an "acid pump" or "proton pump" and examples of PPI's include lansoprazole®, omeprazole®, and pantoprazole®. PPIs rapidly degrade in acidic environments and therefore oral delivery of these compounds is challenging because the gastric pH is very acidic (typically between about pH 1.5 and 1.9). Under gastric conditions, PPIs typically degrade and are not readily available for uptake without being protected.

Due to the pH sensitivity of PPIs, they typically are administered in a form that protects the drug from the acidic gastric environment. Enteric coatings are probably the most widely used method of protecting acid-labile drugs (such as PPIs) from gastric degradation. Enteric coating methods typically form a barrier around drug particles, or an entire dosage form containing a PPI, with a coating that does not dissolve upon introduction to the low pH of the gastric environment. Such enteric coatings typically dissolve at a pH greater than 6, such as that found in the upper small intestine where the PPI is released in an environment where it will not significantly degrade, and therefore can be absorbed. Unfortunately, enteric coated compositions are difficult to formulate as liquids, thus creating difficulty in administration to pediatric patients or patients having difficulty swallowing.

Notwithstanding the above, attempts have been made to formulate liquid dosage forms of acid-labile drugs. However, these formulations generally are made on a single dose basis for administration promptly upon formulation. For example, U.S. Patent No. 5,840,737 recommends dissolving the contents of commercially available capsules containing enterically coated pellets of a PPI in a solution of sodium bicarbonate buffer. In practice, the above method requires a practitioner to open a capsule and release the enterically coated PPI into the buffer. After the contents of the capsule and buffer are combined, the mixture is swirled or mixed for approximately 20 to 30 minutes so that the enteric coating around the PPI dissolves due to the buffer's relatively high pH. As mentioned above, such formulations are for prompt administration of the entire formulation. Moreover, these formulations are typically made and administered in a hospital setting and are not intended for patients that have difficulty swallowing and need a longer term dosing regimen outside of a hospital setting. Pediatric patients experiencing gastrointestinal disorders are one segment of that population.

Several properties are required of a liquid formulation of a PPI intended for patients that have difficulty swallowing a solid dosage form and require a long term dosing regimen outside of a clinical setting. For example, the formulation should maintain the integrity of the active ingredient on a long-term basis. Moreover, the formulation should be able to homogeneously maintain the dispersion of the drug throughout the entire formulation to insure dose uniformity throughout the entire dosing regimen. Specifically, if the liquid formulation contained thirty doses, the concentration of the active ingredient in the first dose of the formulation should be relatively close to the concentration of the active ingredient in the last dose of the formulation. Unfortunately, liquid formulations of a PPI that meet the above requirements are difficult to produce and are not currently available.

There is therefore a need for a liquid formulation of a PPI that maintains the integrity (and therefore efficacy) of the active component for long term use, is easily administered to patients who have difficulty swallowing, is palatable, and can be provided as a bulk suspension from which multiple and uniform doses can be dispensed.

### Summary of the Invention

The present invention provides compositions or formulations comprising a PPI; and a liquid vehicle having a pH greater than 6.5 and a viscosity sufficient to maintain a uniform suspension of the PPI. The formulations can be provided as a kit comprising: a first container having dry ingredients including a PPI; and a second container having liquid ingredients. When the first and second containers are combined they provide a formulation comprising a PPI and a liquid vehicle having a pH greater than 6.5 and a viscosity sufficient to maintain a uniform suspension of the PPI. Advantageously, the bulk formulation requires very little mixing in order for the PPI to become homogenously suspended in the liquid vehicle.

### Detailed Description of the Invention

The present invention provides a liquid formulation of a PPI that is easily administered, maintains the integrity of the PPI, and can be provided in a bulk suspension from which uniform doses can be dispensed. As a result, those who experience difficulty swallowing solid dosage forms may dispense and take single doses of the bulk formulation while being assured that an appropriate amount of the active ingredient is contained in any particular dose. Moreover, no particular expertise is required to dose single aliquots from the bulk formulation and therefore these formulations are ideal for non-clinical settings. The formulation generally comprises a PPI and a liquid vehicle having a pH greater than 6.5. Typically, the liquid vehicle will have a viscosity sufficient to uniformly suspend the PPI for 15 minutes.

PPIs typically are substituted benzimidazole compounds and are well known in the art and may include any of the well known and commercially available PPIs. For example, lansoprazole®, omeprazole®, pantoprazole®, pariprazole®, and leminoprazole® are all suitable for use in the formulation. The amount of PPI in the formulation is a matter of choice that largely is dependent upon the amount of PPI desired for a given dose and the total volume of the formulation. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, and rate of excretion of the specific compound employed; the duration of the treatment; drugs used in combination or coincidental with the specific compound employed; and other factors known to those of ordinary skill in the medical arts. For example, it is well within the skill of the art to start doses of the compound at levels lower than required to achieve the desired therapeutic effect and to gradually increase the dosage until the desired effect is achieved.

Formulations of the invention are administered and dosed in accordance with sound medical practice, taking into account the clinical condition of the individual patient, the site and method of administration, scheduling of administration, and other factors known to medical practitioners.

"Therapeutically effective amounts or dose levels" for purposes herein thus can readily be determined by such considerations as are known in the art. The amount should be effective to achieve improvement, including but not limited to, raising of gastric pH, reduced gastrointestinal bleeding, reduction in the need for blood transfusions, improved survival rate, more rapid recovery, and/or improvement/elimination of symptoms and other indicators as are selected as appropriate measures by those skilled in the art.

As mentioned above, the composition also comprises a liquid vehicle that has a pH of 6.5 or greater, more preferably, a pH of 7.0 or greater, and most preferably a pH of 7.5 or greater. Typically, the pH of a liquid typically will not be greater than 11.5. Liquid vehicles may intrinsically have a pH in the desired range. However, in cases where a liquid does not have the desired pH, the liquid can be modified using pharmaceutically acceptable acids, bases, or other well known and pharmaceutically acceptable means to achieve the desired pH. Preferably, the pH of the liquid vehicle is achieved using buffering salts of any of the well known pharmaceutically acceptable metal salts commonly used to buffer liquids. "Pharmaceutically acceptable" as used herein includes moieties or compounds that are, within the scope of sound medical judgment, suitable for use in contact with the tissues of humans and lower animals without undue toxicity, irritation, allergic response, and the like, and are commensurate with a reasonable benefit/risk ratio. Metal salts typically employed as buffers include but are not limited to phosphates, carbonates, citrates, acetates, hydroxides, tartarates, and silicates of, for example, magnesium, potassium, sodium, aluminum and the like. Examples of metal salts suitable for use in the formulation include, but are not limited to sodium bicarbonate, sodium carbonate, sodium citrate, calcium gluconate, disodium hydrogen phosphate, dipotasium hydrogen phosphate, tripotasium phosphate, sodium tartarate, sodium acetate, calcium glycerophosphate, magnesium hydroxide, aluminum hydroxide, dihydroxy aluminum sodium carbonate, calcium carbonate, aluminum phosphate, aluminum carbonate, dihydroxy aluminum amino acetate, magnesium oxide, magnesium trisilicate, and magnesium carbonate. Other pharmaceutically acceptable buffering agents, such as for example meglamine and tromethamine are also suitable for use in the liquid vehicle and, of course, combinations of any of the foregoing are also suitable.

The amount of the buffering agent in the liquid vehicle may vary widely as long as it is provided in sufficient amount to maintain the pH at a desired level to thereby protect the PPI from degradation in the bulk formulation. Additionally, the liquid vehicle should protect the PPI in the gastric environment sufficiently to allow a therapeautically effective amount of the PPI to reach its intended abosorbtion site in the small intestine. While the amount of the buffering agent in the composition can vary widely and is dependent on various factors such as those mentioned above, the ratio of buffering agent to PPI in the composition typically is between 2:1 and 60:1, preferably between 5:1 and 30:1, more preferable between 8:1 and 20:1.

The liquid vehicle also serves to maintain the homogeneity of the PPI in the formulation that, in large part, is due to the viscosity of the liquid vehicle. Typically, the viscosity is such that after a brief mixing of the bulk solution, the PPI will be homogenously dispersed in the liquid vehicle for a sufficient time to dispense a homogeneous dose of the formulation. A so-called "brief mixing" would include any swirling, shaking, or otherwise agitation, of the contents of the formulation for at least 30 seconds, more preferably at least 60 seconds, and more preferably at least 90 seconds. It is also preferable that the viscosity of the liquid vehicle maintains the homogeneity of the PPI in the liquid vehicle for at least 5 minutes, preferably at least 10 minutes, and most preferably at least 15 minutes. The "homogeneity of the PPI in the formulation", or "a homogenous dispersion of the PPI in the formulation", or a "uniform suspension of the PPI", as well as similar phraseology used herein, means that at least 90% of the intended concentration, and more preferably at least 95% of the intended concentration of the PPI is present throughout the formulation. Thus, for example, if the concentration of the PPI in a particular formulation were 100 mg/ml, a sampling of a milliliter of the bulk formulation at any particular site in the container should contain at least 90 mg of the PPI. Liquids having viscosities of greater than 50 cP, more preferably greater than 60 cP, more preferably greater than 70 cP, and most preferably greater than 100 cP are suitable for the above purposes. The viscosities above are based upon a Brookfield viscometer equipped with #2 spindle rotating at 150 rpm. Additionally, several commercially available liquid antacid formulations have been found as suitable liquid vehicles for use in the formulation provided herein. For example, Alternagel®, Mylanta®, Maalox®, Riopan®, Milk of Magnesia®, Gaviscon®, and the like are suitable liquid vehicles.

In cases where a liquid vehicle is not suitably viscous, various pharmaceutically acceptable additives for increasing the viscosity of a liquid can be employed. For example, sugars such as glucose, sucrose, mannitol and the like; thickening agents such as xanthan gum, guar gum, and gelatin; cellulose derivatives such as methyl cellulose, ethyl cellulose, hydroxypropyl cellulose, and hydroxypropyl methyl cellulose; carbomers such as polyvinyl pyrollidine, and polyvinyl alcohol; as well as clays such as veegum, bentonite, and hectorite are all suitable means for improving the viscosity of a liquid.

The concentration of a PPI in the liquid vehicle is variable and a matter of choice for one skilled in the art that is in large measure dependent upon the desired volume per dose and the desired concentration per dose. Generally, the concentration of PPI in the liquid vehicle is between 0.1 mg/ml and 90 mg/ml, more preferably between 0.5 mg/ml and 60 mg/ml, and most preferably between 0.5 mg/ml and 30 mg/ml.

Formulations provided herein may also contain a variety of other pharmaceutically acceptable flavorings, excipients, sweeteners, preservatives, and antioxidants.

The formulation can be administered to those who are in need of PPI therapy such as those experiencing any of the maladies that PPIs are indicted for use. For example, patients experiencing gastrointestinal disorders such as acid reflux disease, gastro-esophogeal reflux disease, peptic or duedonal ulcers, Zollinger Ellison Syndrome can be treated to alleviate such disorders by administering the formulation of the present invention to such patients. Depending upon factors already mentioned dosing regimens generally will be in the range of between 5 mg and 300 mg of the PPI per day.

Bulk formulations may be provided in a single container having at least 2 individual doses, and typically between 2 and 50 doses of the formulation that can be dispensed individually from the bulk formulation. The formulation may also be provided as a kit having separate containers of the components employed to make the bulk formulation. For example, such a kit may comprise a container having dry ingredients, such as the PPI in various forms, and a container having liquid ingredient of the formulation. The separate containers may be mixed prior to use to generate the complete formulation. In any case, instructions for dosing and mixing may also be provided with such containers of bulk formulation or kits used to form the bulk formulation.

The following examples are provided to further illustrate the present invention and not intended to limit the invention.

### Examples

### Example 1

### PPI Stability in Liquid Vehicles

In this example, the stability of lansoprazole in various liquid vehicles was analyzed.

150 mg of bulk lansoprazole (Takeda Chemical Ind.; Osaka Japan) was added to 50 ml of each of the following liquids: Milk of Magnesia, Mylanta, Maalox, and Alternagel to form a 3mg/ml suspension of the drug. The suspensions were shaken for less than 30 seconds before an initial sample was taken from each container. The concentration of lansoprazole in the initial samples was determined by HPLC. The suspensions were stored in light resistant containers for 31 days at room temperature. At days 10, 23, and 31 after the various suspensions were made, samples were taken after shaking as above and the concentration of lansoprazole in the samples determined as above. The results of this example are shown in Table 1, below.

**Table 1**

| Sample | Initial Lansoprazole Concentration (mg/ml) | Day 10 Lansoprazole Concentration (mg/ml) | Day 23 Lansoprazole Concentration (mg/ml) | Day 31 Lansoprazole Concentration (mg/ml) | Day 31 % of Initial Lansoprazole |
|---|---|---|---|---|---|
| Milk of Magnesia | 3.17 | 3.17 | 3.15 | 3.10 | 97.23 |
| Mylanta | 3.08 | 3.04 | 3.01 | 2.97 | 96.43 |
| Maalox | 3.04 | 3.00 | 2.94 | 2.93 | 96.38 |
| Alternagel | 3.00 | 2.95 | 2.93 | 2.93 | 97.06 |

As shown by Table 1, very little of the lansoprazole was lost after 31 days of storage at room temperature in the various liquid vehicles. Additionally, the drug was suspended uniformly with minimal mixing.

### Example 2

### PPI Stability in various Liquid Vehicles

Similarly to Example 1, in this example, the stability of lansoprazole was assessed in various liquids. Enteric coated pellets form the equivalent of 6 or 18 Prevacid® (TAP Pharmaceutical Products Inc., Lake Forest, IL) capsules was added to 90 ml of the following liquids; Riopan Plus, Gaviscon, Mylanta Supreme, 8.4% NaHCO₃, and a prepared liquid vehicle containing 18.75 gm calcium carbonate, 3.0 gm sodium citrate, 6.0 gm sucrose, 0.5 gm xanthan gum, 0.75 gm peach flavor, and 0.015gm propyl paraben in 150 ml of distilled water, to form 2 mg/ml and 6 mg/ml suspensions of lansoprazole. All of the various solutions were made in duplicate. Once the enteric coatings were dissolved from the pellets, the various suspensions were shaken for 30 seconds before an initial sample was taken to determine the percent of the intended concentration (mg/ml) of lansoprazole in the sample, as determined by HPLC. One of each of the suspensions was stored at 2-8°C and the other suspension was stored at room temperature in light resistant containers. After 28 days of storage the suspensions were briefly (less than 30 seconds) shaken before a sample was taken and the % mg/ml of the intended concentration of lansoprazole was determined using HPLC. The results of this experiment are shown below in Table 2.

**Table 2**

| Suspension Liquid | Intended Lansoprazole Concentration (mg/ml) | Initial Measured Lansoprazole Concentration as a Percentage of Intended Lansoprazole Concentration | Measured Lansoprazole Concentration at 28 Days in 2-8°C as a Percentage of Intended Lansoprazole Concentration | Measured Lansoprazole Concentration at 28 Days in Room Temp, as a Percentage of Intended Lansoprazole Concentration |
|---|---|---|---|---|
| Riopan Plus | 6 | 100.5 | 100.1 | 98.9 |
| Riopan Plus | 2 | 100.5 | 99.9 | 96.5 |
| Gaviscon | 6 | 98.8 | 100.6 | 97.2 |
| Gaviscon | 2 | 100.5 | 99.3 | 95.8 |
| Mylanta Supreme | 6 | 101.0 | 101.6 | 98.4 |
| Mylanta Supreme | 2 | 102.2 | 102.7 | 99.0 |
| 8.4% NaHCO₃ | 6 | 99.5 | 95.8 | 94.7 |
| 8.4% NaHCO₃ | 2 | 98.3 | 91.4 | 80.6 |
| CaCO₃ Solution | 6 | 99.8 | 100.0 | 98.1 |
| CaCO₃ Solution | 2 | 100.1 | 101.3 | 97.3 |

As shown by Table 2, lansoprazole was very stable in most of the liquids tested, in as much as at least 90% of the intended concentration of lansoprazole was measured after 28 days of storage in the various conditions.

### Example 3

### Dose Uniformity of PPI in a Liquid Vehicle

In this example, the ability of a liquid vehicle to uniformly disperse lansoprazole was analyzed. The contents of ten 30 mg Prevacid capsules (TAP Pharmaceutical Products Inc.; Lake Forest, IL) were emptied into 50 ml of Mylanta in a 250 ml graduated cylinder to form a 6 mg/ml suspension. The cylinder was covered and shaken every 5 minutes for 40 minutes. The same Prevacid and Mylanta suspension was formed in a separate 250 ml graduated cylinder and covered. This suspension was shaken on a less regular basis (every 5-10 minutes) for 40 minutes. After the last shaking of the suspensions, 5 ml aliquots (or simulated doses) were poured from the cylinder. The first, fifth and tenth doses were tested by HPLC to determine the concentration of lansoprazole in each dose. The percent of the intended 6mg/ml concentration of lansoprazole in each HPLC tested dose is presented in Table 3, below.

**Table 3**

| Dose | % of Intended Lansoprazole Concentration of Regularly Shaken Suspension | % of Intended Lansoprazole Concentration of Sporadically Shaken Suspension |
|---|---|---|
| 1 | 100.7 | 99.6 |
| 5 | 101.2 | 99.6 |
| 10 | 101.0 | 99.6 |

As shown above, the PPI was uniformly suspended and therefore produced uniform doses throughout the entire bulk formulation.

### Example 4

### Dose Uniformity of PPI's in Liquids of Varying Viscosities

A purpose of this example was to determine whether there is a correlation between the viscosity of a liquid vehicle and the settling rate of a PPI. Various suspensions were formed by disintegrating lansoprazole microgranules (sachet formulations of Prevacid®) in Mylanta, Gaviscon, and an 8.4% NaHCO₃ solution. The viscosities of the various suspensions were measured using a Brookfield viscometer. Additionally, the settling of the lansoprazole in the suspensions was determined by taking a sample from the top, middle and bottom of the graduated cylinders containing the suspensions after various time periods. These samples were then analyzed by HPLC to determine the percent of the intended concentration in each of these samples.

1200 ml of Gaviscon, Mylanta, and the 8.4% NaHCO₃ were placed in three separate 2000 ml screw cap Erlenmeyer flasks. 88.8 gm of lansoprazole microgranules were then added to each of the flasks and shaken for 30 minutes to disintegrate the microgranules. A 5 ml aliquot was then removed from each of the flasks after each flask was vigorously shaken. This sample was then prepared for HPLC analysis and was used as a control for each of the subsequent samplings from the flasks. The control concentration of lansoprazole in Mylanta was 5.21 mg/ml, the control concentration of lansoprazole in Gaviscon was 5.26 mg/ml and the control concentration of lansoprazole in NaHCO₃ was 5.05 mg/ml.

250 ml aliquots of each flask were then poured into 4 separate 250 ml graduated cylinders such that there were four 250 ml samples from each of the different flasks. The samples in the graduated cylinders were then used to evaluate settling times. Specifically, the contents of one cylinder from each of the different flasks was sampled after 5, 10, 15, and 30 minutes from the time the cylinders were allowed to stand undisturbed. Samples (5 ml) were taken from the top middle and bottom of the cylinders and prepared for HPLC analysis.

The percent of recovered lansoprazole in each sample of a given time period from the Mylanta suspension is shown in Table 4, below

**Table 4**

| Settling Time | Top | Middle | Bottom |
|---|---|---|---|
| 5 | 99.6 | 100.2 | 100.2 |
| 10 | 100.6 | 100.0 | 100.0 |
| 15 | 100.4 | 99.6 | 100.4 |
| 30 | 100.6 | 100.2 | 100.0 |

The percent of recovered lansoprazole in each sample of a given time period from the Gaviscon suspension is shown in Table 5, below

**Table 5**

| Settling Time | Top | Middle | Bottom |
|---|---|---|---|
| 5 | 98.5 | 97.3 | 97.9 |
| 10 | 98.1 | 97.3 | 97.3 |
| 15 | 100.2 | 99.4 | 98.1 |
| 30 | 100.2 | 97.3 | 98.7 |

The percent of recovered lansoprazole in each sample of a given time period from the NaHCO₃ suspension is shown in Table 6, below

**Table 6**

| Settling Time | Top | Middle | Bottom |
|---|---|---|---|
| 5 | 98.8 | 98.6 | 98.0 |
| 10 | 93.5 | 96.0 | 106.7 |
| 15 | 81.2 | 97.6 | 105.3 |
| 30 | 18.8 | 89.3 | 124.8 |

The viscosities of the various suspension liquids and suspension liquids containing the lansoprazole were also determined. The viscosity measurements were taken with a Brookfield viscometer equipped with a number 2 spindle that was set at various speeds. The viscosity measurements were taken using 150 ml of the various liquids in a 200 ml tall-form beaker at room temperature. Generally, viscosity measurements are most accurate when the % scale reading is above 10. The viscosities recorded as above, are presented in Table 7, below.

**Table 7**

| Liquid | Spindle Speed (rpm) | % Scale | Viscosity (cP) |
|---|---|---|---|
| 8.4% NaHCO₃ | 200 | 3.9 | 5.8 |
| 8.4% NaHCO₃ (with lansoprazole) | 200 | 8.8 | 13.2 |
| Mylanta | 100 | 25 | 76 |
| | 150 | 37 | 74 |
| | 200 | 50 | 75 |
| Mylanta (with lansoprazole) | 100 | 44 | 132 |
| | 150 | 65 | 130 |
| Gaviscon | 2 | 37 | 5590 |
| | 5 | 54 | 3160 |
| | 10 | 67 | 2010 |
| Gaviscon (with lansoprazole) | 2 | 22 | 3250 |
| | 5 | 54 | 1930 |
| | 10 | 46 | 1360 |
| | 15 | 57 | 1130 |

Based upon the data above, the ability of a liquid vehicle to maintain the uniformity of a PPI in a bulk liquid dosage form is best when the viscosity of the formulation is greater than 13.2.

## Claims

1. A composition comprising;
(a) a proton pump inhibitor; and
(b) a liquid vehicle having a pH greater than 6.5 and a viscosity of at least 50cP.

2. The composition of claim 1 wherein the liquid vehicle comprises a metal salt buffer.

3. The composition of claim 1 wherein the proton pump inhibitor is lansoprazole.

4. The composition of claim 1 wherein the composition has at least 2 doses of the proton pump inhibitor.

5. The composition of claim 1 wherein the viscosity of the composition is greater than 50 cP.

6. The composition of claim 1 wherein the viscosity of the composition is greater than 70 cP.

7. A composition comprising a proton pump inhibitor and a liquid vehicle having a pH greater than 6.5 and a viscosity of at least 50cP for use in a method of treating a patient with a gastrointestinal disorder.

8. A kit comprising:
(a) a first container having dry ingredients including a proton pump inhibitor; and
(b) a second container having liquid ingredients;
wherein when said first and second container are combined they provide a formulation comprising a proton pump inhibitor and a liquid vehicle having a pH greater than 6.5 and a viscosity of at least 50cP.

## Patentansprüche

1. Eine Zusammensetzung, die Folgendes umfasst:
(a) einen Protonenpumpeninhibitor; und
(b) ein flüssiges Vehikel mit einem pH-Wert von größer als 6,5 und einer Viskosität von mindestens 50cP.

2. Die Zusammensetzung gemäß Anspruch 1, worin das flüssige Vehikel einen Metallsalzpuffer umfasst.

3. Die Zusammensetzung gemäß Anspruch 1, worin der Protonenpumpeninhibitor Lansoprazol ist.

4. Die Zusammensetzung gemäß Anspruch 1, worin die Zusammensetzung mindestens 2 Dosen des Protonenpumpeninhibitors hat.

5. Die Zusammensetzung gemäß Anspruch 1, worin die Viskosität der Zusammensetzung größer als 50 cP ist.

6. Die Zusammensetzung gemäß Anspruch 1, worin die Viskosität der Zusammensetzung größer als 70 cP ist.

7. Eine Zusammensetzung, die einen Protonenpumpeninhibitor und ein flüssiges Vehikel mit einem pH-Wert von größer als 6,5 und einer Viskosität von mindestens 50cP hat, zur Verwendung in einem Verfahren zur Behandlung eines Patienten mit einer gastrointestinalen Erkrankung.

8. Ein Kit, das Folgendes umfasst:
(a) einen ersten Behälter mit trockenen Inhaltsstoffen einschließlich eines Protonenpumpeninhibitors; und
(b) einen zweiten Behälter mit flüssigen Inhaltsstoffen;
worin der erste und der zweite Behälter, wenn sie kombiniert werden, eine Formulierung bereitstellen, die einen Protonenpumpeninhibitor und ein flüssiges Vehikel mit einem pH-Wert von größer als 6,5 und einer Viskosität von mindestens 50cP umfasst.

## Revendications

1. Composition comprenant :
(a) un inhibiteur de la pompe à protons ; et
(b) un véhicule liquide ayant un pH supérieur à 6,5 et une viscosité d'au moins 50 cP.

2. Composition selon la revendication 1, dans laquelle le véhicule liquide comprend un tampon sel métallique.

3. Composition selon la revendication 1, dans laquelle l'inhibiteur de la pompe à protons est le lansoprazole.

4. Composition selon la revendication 1, laquelle composition a au moins 2 doses de l'inhibiteur de la pompe à protons.

5. Composition selon la revendication 1, dans laquelle la viscosité de la composition est supérieure à 50 cP.

6. Composition selon la revendication 1, dans laquelle la viscosité de la composition est supérieure à 70 cP.

7. Composition comprenant un inhibiteur de la pompe à protons et un véhicule liquide ayant un pH supérieur à 6,5 et une viscosité d'au moins 50 cP, pour utilisation dans une méthode de traitement d'un patient ayant un trouble gastro-intestinal.

8. Trousse comprenant :
(a) un premier récipient ayant des ingrédients secs comprenant un inhibiteur de la pompe à protons ; et
(b) un deuxième récipient ayant des ingrédients liquides ;
dans laquelle, quand lesdits premier et deuxième récipients sont combinés, ils forment une formulation comprenant un inhibiteur de la pompe à protons et un véhicule liquide ayant un pH supérieur à 6,5 et une viscosité d'au moins 50 cP.
